# FASCICULE DE BREVET EUROPEEN

(11) **EP 4 311 860 B1**
(45) Date de publication et mention de la délivrance du brevet: **09.12.2026**
(21) Numéro de dépôt: 23187466.0
(22) Date de dépôt: 25.07.2023
(51) Int. Cl.: C12Q 1/6806

(54) **MÉTHODE D'ÉVALUATION IN VITRO DE L'ACTIVITÉ PHOTOPROTECTRICE D'UN ACTIF**
VERFAHREN ZUR IN VITRO BEURTEILUNG DER LICHTSCHUTZAKTIVITÄT EINES WIRKSTOFFS
METHOD FOR IN VITRO EVALUATION OF THE PHOTOPROTECTIVE ACTIVITY OF AN ACTIVE INGREDIENT

(30) Priorité: 25.07.2022 FR 2207631
(43) Date de publication de la demande: 31.01.2024
(73) Titulaire: Pierre Fabre Dermo-Cosmétique, 81500 Lavaur (FR)
(72) Inventeur: BACQUEVILLE, Daniel, 81106 CASTRES (FR); DUPLAN, Hélène, 81106 CASTRES (FR); BESSOU-TOUYA, Sandrine, 81106 CASTRES (FR); DOUKI, Thierry, 38000 GRENOBLE (FR); CAILLAT Sylvain, 38210 TULLINS (FR); REYNAUD, Noémie, 69100 VILLEURBANNE (FR)
(74) Mandataire: Regimbeau

(56) Documents cités:
- YOUNG A ET AL: "Sub-optimal Application of a High SPF Sunscreen Prevents Epidermal DNA Damage in Vivo", ACTA DERMATO-VENEREOLOGICA., vol. 98, no. 9, 1 January 2018 (2018-01-01), United Kingdom, pages 880 - 887, XP093049277, ISSN: 0001-5555, DOI: 10.2340/00015555-2992
- DOUKI THIERRY: "The variety of UV-induced pyrimidine dimeric photoproducts in DNA as shown by chromatographic quantification methods", vol. 12, no. 8, 1 August 2013 (2013-08-01), GB, pages 1286 - 1302, XP093050360, ISSN: 1474-905X, Retrieved from the Internet <URL:https://link.springer.com/content/pdf/10.1039/c3pp25451h.pdf> DOI: 10.1039/c3pp25451h
- BACQUEVILLE D ET AL: "Comparative analysis of solar radiation-induced cellular damage between ex vivo porcine skin organ culture and in vitro reconstructed human epidermis", INTERNATIONAL JOURNAL OF COSMETIC SCIENCE, KLUWER ACADEMIC PUBLISHERS, DORDRECHT, NL, vol. 31, no. 4, 20 May 2009 (2009-05-20), pages 293 - 302, XP071469506, ISSN: 0142-5463, DOI: 10.1111/J.1468-2494.2009.00506.X
- BACQUEVILLE D. ET AL: "), an innovative sun filter with unique optical properties", JEADV , THE JOURNAL OF THE EUROPEAN ACADEMY OF DERMATOLOGY AND VENEREOLOGY, vol. 36, no. S6, 23 June 2022 (2022-06-23), NL, pages 29 - 37, XP093127184, ISSN: 0926-9959, Retrieved from the Internet <URL:https://onlinelibrary.wiley.com/doi/full-xml/10.1111/jdv.18196> DOI: 10.1111/jdv.18196
- BACQUEVILLE D ET AL: "A new hair follicle-derived human epidermal model for the evaluation of sunscreen genoprotection", JOURNAL OF PHOTOCHEMISTRY AND PHOTOBIOLOGY B: BIOLOGY, ELSEVIER SCIENCE S.A., BASEL, CH, vol. 151, 19 June 2015 (2015-06-19), pages 31 - 38, XP029278685, ISSN: 1011-1344, DOI: 10.1016/J.JPHOTOBIOL.2015.06.015
- BACQUEVILLE D. ET AL: "Phenylene Bis-Diphenyltriazine (TriAsorB), a new sunfilter protecting the skin against both UVB?+?UVA and blue light radiations", PHOTOCHEMICAL & PHOTOBIOLOGICAL SCIENCES, vol. 20, no. 11, 1 November 2021 (2021-11-01), GB, pages 1475 - 1486, XP055977571, ISSN: 1474-905X, Retrieved from the Internet <URL:https://link.springer.com/content/pdf/10.1007/s43630-021-00114-x.pdf> DOI: 10.1007/s43630-021-00114-x

## Description

### DOMAINE DE L'INVENTION

Le domaine de l'invention concerne l'évaluation de l'efficacité de la photoprotection de l'ADN évaluée *in vitro* par la quantification des produits de réparation de l'ADN.

La surexposition au rayonnement ultraviolet (UV) qu'il soit solaire ou artificiel a des effets délétères majeurs sur les systèmes vivants. Chez l'homme, les UV sont responsables du photo-vieillissement, de l'érythème, de l'immunosuppression et des cancers de la peau (Huang et al., 2020, Curr. Dermatol. Rep. 9(1), 22-9 ; Subhadarshani et al. 2020, Curr Dermatol Rep. 9(3) : 189-99). La capacité des UV à induire des tumeurs cutanées est principalement expliquée par leurs propriétés génotoxiques et mutagènes (Cadet and Douki, 2018, Photochem. Photobiol. Sci. 17 : 1816-1841). Un certain nombre de preuves expérimentales révèle que les UVB (280-320 nm) constituent la partie la plus nocive du spectre solaire (Marzouki et al., 2017, Life Sci. 188 : 10-16). L'absorption dans cette gamme de longueur d'onde induit des photoréactions dans l'ADN et conduit à la formation de dimères de pyrimidine. Il s'agit notamment des dimères de cyclobutane pyrimidine (CPD) et les photoproduits pyrimidine (6-4) pyrimidone (64PP) et leurs isomères de valence Dewar. Ces photoproduits peuvent se former aux quatre séquences bipyrimidines possibles (TT, TC, CT et CC) mais en proportion différente. Les UVA sont au moins vingt fois plus intenses que les UVB mais sont deux à trois ordres de grandeur moins efficaces pour endommager l'ADN. Par conséquent, l'accent a été mis sur les dimères de pyrimidine induits par les UVB pour les études *in vitro* et *in vivo.* Ces photoproduits de l'ADN participent à l'initiation du processus tumoral comme le montre la signature mutationnelle spécifique des carcinomes basaux et squameux (Bonilla et al., 2016, Nat. Gen. 48(4) : 398-406), et du mélanome (Hodis et al.,2012, Cell 150(2) : 251-263). Les photoproduits d'ADN induits par les UV sont éliminés du génome par la voie de réparation de l'excision de nucléotides sous la forme d'un fragment d'oligonucléotides monocaténaires de 25 à 30 bases de long portant les dommages.

L'induction de photoproduits de l'ADN par la lumière du soleil dans les kératinocytes et les mélanocytes est un évènement clé dans l'induction de mutations conduisant à des carcinomes cutanés et des mélanomes. La prévention de la formation de cette classe de dommages à l'ADN est donc essentielle à la photoprotection, comme en témoigne le lien sans ambiguïté entre l'utilisation correcte des produits solaires et le risque de cancer (Green et al., 1999, Lancet 354 : 723-729).

Le facteur de protection solaire utilisé pour étiqueter les produits de photoprotection est une valeur pratique et fiable, mais il est basé sur la prévention de l'érythème. Le facteur de protection n'est pas nécessairement le même pour les autres réponses photobiologiques. En effet, les produits peuvent avoir le même facteur de protection solaire mais leurs propriétés physiques et d'absorbance peuvent différer, ce qui entraine des différences dans l'absorption des chromophores responsables de l'immunosuppression, des dommages à l'ADN ou de la pigmentation. De plus, les évènements biologiques sous-jacents à l'érythème et à l'initiation du cancer sont complètement différents. L'évaluation des propriétés photoprotectrices des produits solaires nécessite donc l'évaluation directe de la protection qu'ils offrent à l'ADN.

Certains travaux ont abordé la question de l'évaluation de la photoprotection de l'ADN dans les études cliniques impliquant l'exposition de volontaires (Young et al., 2018, Acta Dermato-Venereologica, 98 : 880-887). Le rapport entre le niveau de dommages dans la peau exposée à la lumière du soleil simulée en l'absence ou la présence du produit solaire fournit un facteur de protection de l'ADN. Cette approche est entravée par des considérations éthiques car elle implique la quantification de photoproduits dans l'ADN des biopsies cutanées. D'autres techniques sont plus faciles à mettre en place, telle que la collecte de bulles de succion (Josse et al., 2018, J. Photochem. Photobiol. B. 179 : 1-6), mais elles restent invasives et nécessitent toujours l'exposition de volontaires. Les modèles *in vitro* tels que les explants de peau humaine ou les peaux reconstituées, apparaissent comme des alternatives intéressantes (Arase and Jung 1986, Photodermatol. 3 : 56-59 ; Bacqueville D et Mavon A, 2009, Int. J. Cosmet Sci. Aug;31(4):293-302 ; Bacqueville et al., 2015, J. Photochem. Photobiol. B. 151 : 31 -38) ; Bacqueville D. et al., 2021, Photochem. Photobiol. Sci., Nov;20(11):1475-1486 ; Bacqueville D. et al., 2022, J. Eur. Acad. Dermatol. Venereol. Jun;36 Suppl 6:29-37.

Des résultats récents issus de la littérature suggèrent que des dimères de pyrimidine seraient détectables sous forme libre dans des explants cutanés, sans doute suite à leur élimination par les systèmes de réparation (Choi et al. (2020) DNA Repair 86, 102766. 10.1016/j.dnarep.2019.102766). Plus récemment, des CPD inclus dans des grands fragments d'ADN liés à des protéines et localisés dans des vésicules extracellulaires ont été retrouvés dans le milieu de kératinocytes humains en culture (Carpenter et al., 2022 J. Invest. Dermatol. In press DOI: 10.1016/j.jid.2022.04.030).

Cependant, il n'est à ce jour pas possible de détecter les dimères de pyrimidine dans le milieu de culture de modèles de peau reconstituée.

Sur la base de ces observations, les inventeurs ont mis en évidence, de façon inattendue, qu'il était possible de développer une approche analytique pour quantifier des produits de réparation de l'ADN libérés dans le milieu de culture de modèles in *vitro.* Cette méthode permet l'évaluation de l'activité photoprotectrice d'un actif photoprotecteur.

### RESUME DE L'INVENTION

La présente description fournit donc une méthode d'évaluation de l'activité photoprotectrice d'un actif photoprotecteur, la méthode comprenant :
a) la mise en contact de l'actif avec un substitut cutané, puis
b) L'exposition du substitut cutané à un rayonnement, puis
c) Le dosage de dimères de pyrimidine dans le substitut cutané et/ou son milieu de culture.

Plus particulièrement, la méthode décrite ici est caractérisée en ce le dosage comprend une digestion enzymatique d'acides nucléiques et un dosage des dimères de pyrimidine par chromatographie liquide.

De façon encore plus préférée, le dosage comprend des étapes dans lesquelles :
- les acides nucléiques contenus dans le substitut cutané et/ou son milieu de culture sont extraits en phase solide ; puis
- l'éluat contenant les acides nucléiques obtenu suite à l'étape d'extraction en phase solide est concentré ; puis
- les acides nucléiques contenus dans l'éluat concentré ainsi obtenu sont digérés enzymatiquement ; puis
- les dimères de pyrimidine obtenus suite à l'étape de digestion enzymatique sont dosés par chromatographie liquide.

Dans un mode de réalisation particulier, le dosage des dimères de pyrimidine comprend la détermination de la concentration des dimères de pyrimidine dans le substitut cutané et/ou son milieu de culture, avantageusement par spectrométrie de masse.

Préférablement, la méthode comprend une étape supplémentaire de comparaison de la concentration des dimères de pyrimidine dans le substitut cutané et/ou son milieu de culture avec une référence.

Dans un mode de réalisation préféré, la référence est la concentration des dimères de pyrimidine dans un substitut cutané avec lequel l'actif n'a pas été mis en contact et/ou son milieu de culture. Dans un autre mode de réalisation préféré, la référence est la concentration des dimères de pyrimidine dans le substitut cutané et/ou dans le milieu de culture du substitut cutané avant la mise en contact avec l'actif.

Selon un mode de réalisation particulier, les dimères de pyrimidine sont des dimères de thymine, de préférence TT 64PP ou TT CPD.

Selon un mode de réalisation particulier, l'actif est au moins un filtre solaire.

Selon un mode de réalisation particulier, l'actif est appliqué sur le substitut cutané.

Selon un mode de réalisation particulier, l'actif est ajouté directement dans le milieu de culture.

Selon un mode de réalisation particulier, le rayonnement est un rayonnement ultraviolet et/ou lumière bleue haute énergie visible et/ou infrarouge.

Selon un autre aspect, la description a aussi pour objet une méthode d'identification d'un actif photoprotecteur, ladite méthode comprenant :
a) L'évaluation de l'activité photoprotectrice de l'actif selon la méthode décrite ici, et
b) L'identification de l'actif comme un actif photoprotecteur si l'activité photoprotectrice évaluée dans l'étape a) est supérieure à un seuil.

Selon un mode de réalisation particulier, le seuil est l'activité photoprotectrice d'un actif photoprotecteur connu évaluée selon la méthode décrite ici.

### LEGENDE DES FIGURES

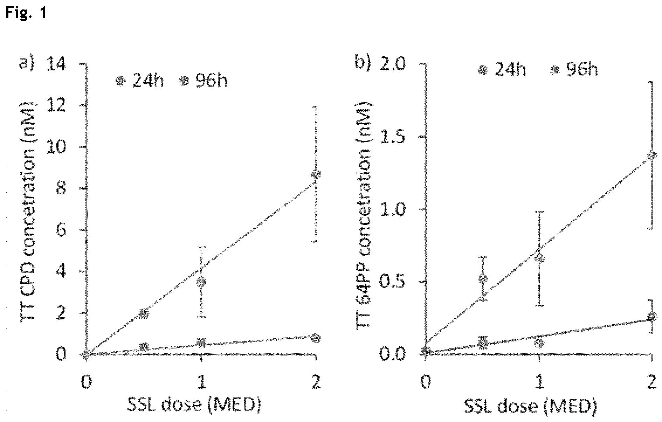

**Figure 1** : Augmentation de la concentration en a) TT CPD et b) TT 64PP dans le milieu d'explants humains prélevés 24 ou 96 h après l'exposition à des doses croissantes de SSL. Les valeurs sont des moyennes ± l'écart-type (n=3).

### DESCRIPTION DETAILLEE

Les inventeurs ont mis au point une nouvelle méthode permettant d'évaluer l'activité photoprotectrice d'actifs qui est fondée sur le dosage des dimères de pyrimidine dans des substituts cutanés et/ou leur milieu de culture. Cette méthode est particulièrement avantageuse parce qu'elle permet de détecter et quantifier aisément les dimères de pyrimidines résultant par exemple d'une irradiation aux ultraviolets. Elle est particulièrement avantageuse parce qu'elle peut être utilisée pour évaluer l'activité photoprotectrice d'actifs. En outre, la présente méthode permet de doser les dimères de pyrimidine directement dans le milieu de culture des substituts cutanés. Il est ainsi possible de suivre au cours du temps la formation de ces dimères, ce qui est particulièrement appréciable pour déterminer, par exemple, l'activité photoprotectrice d'actifs dans la durée.

La description a pour objet une méthode d'évaluation de l'activité photoprotectrice d'un actif photoprotecteur, la méthode comprenant :
a) La mise en contact de l'actif avec un substitut cutané, puis
b) L'exposition du substitut cutané à un rayonnement, puis
c) Le dosage des dimères de pyrimidine dans le substitut cutané et/ou son milieu de culture.

Préférablement, les dimères de pyrimidine sont dosés dans le milieu de culture du substitut cutané. La description a donc plus préférentiellement pour objet une méthode d'évaluation de l'activité photoprotectrice d'un actif photoprotecteur, la méthode comprenant :
a) La mise en contact de l'actif avec un substitut cutané, puis
b) L'exposition du substitut cutané à un rayonnement, puis
c) Le dosage des dimères de pyrimidine dans le milieu de culture du substitut cutané.

Dans un mode de réalisation préféré, le dosage des dimères de pyrimidine comprend des étapes de :
- digestion enzymatique des acides nucléiques ; puis
- dosage des dimères de pyrimidine par chromatographie liquide.

Dans un mode de réalisation encore plus préféré, le dosage des dimères de pyrimidine comprend des étapes dans lesquelles :
- les acides nucléiques contenus dans le substitut cutané et/ou dans le milieu de culture du substitut cutané sont extraits en phase solide ; puis
- l'éluat contenant les oligonucléotides issus de la réparation de l'ADN obtenu suite à l'étape d'extraction en phase solide est concentré ; puis
- les acides nucléiques contenus dans l'éluat concentré ainsi obtenu sont digérés enzymatiquement ; puis
- les dimères de pyrimidine obtenus suite à l'étape de digestion enzymatique sont dosés par chromatographie liquide.

Par « acide nucléique », on entend ici l'ADN et l'ARN, préférablement l'ADN. L'acide nucléique selon la présente description peut être circulaire ou linéaire ; il est préférentiellement linéaire. L'acide nucléique peut aussi être bicaténaire ou monocaténaire. De préférence, l'acide nucléique est monocaténaire. L'acide nucléique peut être sous forme de molécules complètes ou de fragments. Dans un mode de réalisation préféré, l'acide nucléique est un fragment et plus particulièrement un oligonucléotide. Un « oligonucléotide » tel qu'on l'entend ici est une molécule d'acide nucléique qui comprend moins de 50 nucléotides, préférablement moins de 40 nucléotides, encore plus préférablement moins de 30 nucléotides. Un oligonucléotide peut aussi comprendre plus de 15 nucléotides, préférablement plus de 20 nucléotides, encore plus préférablement plus de 25 nucléotides. Enfin, un oligonucléotide tel qu'on l'entend ici peut comprendre entre 15 et 50 nucléotides, préférablement entre 20 et 40 nucléotides, encore plus préférablement entre 25 et 30 nucléotides. Selon un mode de réalisation particulièrement avantageux, l'acide nucléique est un oligonucléotide monocaténaire linéaire tel que, par exemple, les oligonucléotides générés par le mécanisme de réparation de l'ADN à la suite d'un dommage induit par les UV.

La méthode comprend avantageusement une étape supplémentaire de quantification de la concentration des dimères de pyrimidine dans le substitut cutané et/ou son milieu de culture, avantageusement par spectrométrie de masse.

Par « photoproduit », on entend ici tout produit d'une réaction photochimique. Un « photoproduit » au sens de la description est plus particulièrement un dimère de pyrimidine. Par « dimère de pyrimidine », on entend ici une lésion moléculaire de l'ADN résultant d'une réaction photochimique entre des résidus de thymine ou de cytosine adjacents. De façon préférée, les dimères de pyrimidine tels qu'entendus ici sont des dimères thymine. Les dimères de pyrimidine comprennent notamment les dimères de pyrimidine de type cyclobutane (CPD) et les photoproduits pyrimidine (6-4) pyrimidone (64PP). Les dimères de pyrimidine sont avantageusement choisis parmi TT 64PP et TT CPD.

La voie de réparation par excision de nucléotides reconnaît les dommages dans l'ADN résultant d'une exposition aux UV et élimine un fragment de 25 à 30 nucléotides du brin d'ADN contenant la lésion. Les photoproduits dans l'échantillon biologique peuvent être quantifiés par extraction en phase solide des fragments d'ADN générés par la réparation de l'ADN et excrétés. Avantageusement, le procédé comprend une étape préliminaire de synthèse chimique de photoproduits TT marqués par des isotopes stables pour obtenir les étalons internes permettant ainsi par la suite une quantification en spectrométrie de masse en dilution isotopique.

Un protocole analytique spécifique a été développé selon la description. Le protocole se divise en 4 grandes étapes :
- L'extraction des acides nucléiques par extraction en phase solide (SPE) ;
- La concentration des acides nucléiques ;
- La digestion enzymatique des acides nucléiques ;
- Le dosage des dimères de pyrimidine par chromatographie couplée le cas échéant à la spectrométrie de masse.

La première étape est l'extraction en phase solide de l'échantillon biologique (c'est-à-dire le substitut cutané et/ou son milieu de culture), avantageusement après ajout d'étalons internes qui assurent l'aspect quantitatif malgré une préparation d'échantillons complexe. L'extraction en phase solide (en anglais « solid-phase extraction », ou SPE) est une méthode de préparation de l'échantillon au cours de laquelle des composés en solution ou en suspension dans une phase liquide sont séparés des autres éléments du mélange par adsorption sélective sur une phase solide (colonne) en fonction de leurs propriétés physico-chimiques. Cette étape d'extraction en phase solide permet d'isoler les oligonucléotides, y compris les oligonucléotides portant les dimères de pyrimidine, des interférents contenus dans l'échantillon biologique, grâce à la phase solide. La phase stationnaire de la phase solide est avantageusement une phase polymérique ; plus préférentiellement une phase polymérique optimisée pour les oligonucléotides telle qu'une HRX-100. Les oligonucléotides sont absorbés sur la phase stationnaire de la colonne, puis un solvant ayant une force d'élution croissante (ajout de méthanol dans l'éluant) pouvant les éluer permet de les récupérer. Le solvant est avantageusement un mélange hydroalcoolique ; avantageusement eau-méthanol. Le solvant peut également comprendre un tampon, tel que le TEAA (acétate de triéthylammonium). Entre temps, un lavage est effectué afin de nettoyer la colonne des interférents.

Avantageusement, 4 étapes composent la SPE :
1. Conditionnement : Activation de la phase polymérique de la colonne. La colonne peut être conditionnée par un mélange eau-méthanol comprenant de 10% à 20% en volume de méthanol. Le mélange peut comprendre un tampon, tel que le TEAA.
2. Dépôt : Dépôt de l'échantillon
3. Lavage : Elimination des interférents. Le solvant de lavage comprend avantageusement de l'eau et moins de 30% en volume de méthanol. Il peut lui aussi comprendre un tampon, tel que le TEAA.
4. Elution : Récupération des composés. Le solvant d'élution comprend avantageusement au moins 30% en volume de méthanol, avantageusement de 50% à 100% en volume de méthanol. Le solvant peut lui aussi comprendre un tampon, tel que le TEAA.

La seconde étape est la concentration de l'éluat récupéré. La concentration peut être réalisée par évaporation sous vide des solvants organiques, et par l'utilisation d'un lyophilisateur pour permettre l'élimination de l'eau et des sels volatils.

La troisième étape est la digestion enzymatique qui peut être réalisée en suivant les méthodes bien connues. Cette étape permet de libérer des nucléosides monomériques et des dimères de pyrimidine, lesquels pourront ensuite être dosés. Toute enzyme possédant une activité DNAse peut être utilisée dans ce type de réaction, pourvu que l'hydrolyse soit complète. Cependant, il est avantageux d'utiliser une enzyme capable d'hydrolyser toutes les liaisons phosphodiesters des molécules d'acide nucléique. L'hydrolyse pourra donc être réalisée avec une ou plusieurs enzymes dotées d'une activité phosphodiestérase et capable d'agir sur les acides nucléiques présents dans l'éluat. De façon préférée, l'hydrolyse sera réalisée par une combinaison de phosphodiestérase II, DNase II et nucléase P1 à pH acide. A pH basique, on utilisera préférentiellement une combinaison de phosphatase alcaline et de phosphodiestérase I. Il est aussi possible d'alterner les traitements à pH acide et à pH basique. Ainsi, il peut être avantageux pour que la digestion soit bien complète de réaliser une première hydrolyse à pH acide avec une combinaison de phosphodiestérase II, DNase II et nucléase P1, suivie d'une deuxième hydrolyse à pH basique à l'aide d'une combinaison de phosphatase alcaline et de phosphodiestérase I.

L'étape finale est le dosage par chromatographie liquide, couplée le cas échéant à la spectrométrie de masse pour une quantification absolue des photoproduits ciblés. Dans cette optique, il est particulièrement avantageux d'utiliser des standards internes pour assurer l'aspect quantitatif malgré une préparation d'échantillons complexe. L'approche a été optimisée pour limiter les effets de matrice dus à l'échantillon biologique (substitut cutané et/ou son milieu de culture) et améliorer spécificité et sensibilité.

Une étape de SPE en ligne sur une colonne de gel de silice en C18 peut être avantageusement ajoutée. Elle permet d'éliminer une partie des impuretés présent dans les échantillons en début d'élution avant la séparation analytique proprement dite et ainsi d'augmenter la sensibilité de la méthode. Pour l'étape d'analyse par chromatographie liquide, la colonne d'analyse est alors préférablement différente de celle utilisée lors de l'étape de SPE en ligne, par exemple de type pentafluorophényle, apportant ainsi une séparation complémentaire à celle sur gel de silice en C18.

L'analyse par spectrométrie de masse porte avantageusement sur la détection des ions-fragments de TT CDP et TT 64PP. Les ions-fragments correspondant des étalons internes ont été aussi suivis. La concentration en photoproduits a été déterminée en calculant le ratio entre le signal de chaque ion et de son analogue dans les étalons internes à la fois dans les standards et les échantillons.

Par « substitut cutané » on entend une culture de cellules cutanées composée d'au moins une couche. Les substituts cutanés selon la description comprennent notamment les cultures de cellules cutanées en monocouche, les cultures de cellules cutanées en bicouche et les modèles tissulaires, dont les cultures d'épiderme reconstruit ou de peau reconstruite. En effet, comme il est souvent difficile de travailler sur des explants frais, il est particulièrement avantageux, dans le cadre de la présente description, d'utiliser des cultures de cellules cutanées. Par « épiderme reconstruit », on entend un épiderme généré *in vitro* par des techniques classiques bien connues de la personne du métier (cf., par exemple, Limat et Hunziger. Cells Tissues Organs 2002;172:79-85 ; Poumay et al., Arch Dermatol Res. 2004;296(5):203-11). Par « peau reconstruite » on entend une culture contenant au moins deux compartiments : un compartiment dermique, et un compartiment épidermique. De préférence, la composante épidermique de la peau reconstruite contient des kératinocytes. De préférence, la composante dermique contient des fibroblastes.

Avantageusement, les cellules cutanées comprennent des cellules normales, saines ou pathologiques pouvant entrainer des troubles cutanés, ou des cellules issues de lignées. Des cellules cutanées mises en culture peuvent notamment être obtenues à partir d'explant de tissu cutané. Par « explant » ou « explant de peau », on entend ici un prélèvement de cellules ou de tissu cutané, lequel peut être réalisé dans un but chirurgical ou pour effectuer des analyses. En particulier, un explant peut être obtenu lors d'une exérèse chirurgicale. Par « exérèse », on entend ici une intervention chirurgicale consistant à découper (exciser) une partie plus ou moins large ou profonde de la peau pour en traiter une anomalie ou une excroissance. On procède à une exérèse soit pour retirer une tumeur cancéreuse ou suspecte de l'être, soit pour traiter une anomalie bénigne de la peau qui est gênante, que ce soit pour des raisons fonctionnelles ou esthétiques. Une exérèse au sens de la description inclut par exemple les échantillons de peau obtenus après chirurgie plastique (plastie mammaire, abdominale, lifting, prélèvement préputial, otoplastie, c'est-à-dire recollement d'oreille, syndactylie ou doigt surnuméraire, etc.).

Un explant peut aussi être obtenu par biopsie. Par « biopsie », on entend ici un prélèvement de cellules ou tissu cutané réalisé à des fins d'analyse. Plusieurs types de procédures de biopsies sont connus et pratiqués dans le domaine. Les types les plus communs comprennent (1) la biopsie incisionnelle, dans laquelle seul un échantillon du tissu est prélevé, (2) la biopsie excisionnelle (ou biopsie chirurgicale) qui consiste en l'ablation totale d'une masse tumorale, réalisant ainsi un geste thérapeutique et diagnostique, et (3) la biopsie à l'aiguille, dans laquelle un échantillon de tissu est prélevé avec une aiguille, celle-ci pouvant être large ou fine. D'autres types de biopsie existent, comme par exemple le frottis ou le curettage, et sont aussi englobés dans la présente description.

Alternativement, lesdites cellules cutanées peuvent être obtenues par différentiation de cellules souches (Guenou et al., Lancet, 374(9703) : 1745-1753, 2009 ; Nissan et al., Proc. Natl. Acad. Sci., 108(36) : 14861-14866, 2011 ; Kraehenbuehl et al., Nature Methods, 8 : 731-736, 2011). Selon un mode de réalisation, lesdites cellules souches ne sont pas des cellules souches embryonnaires humaines. Si des cellules souches embryonnaires humaines sont utilisées, elles sont obtenues par un procédé ne nécessitant pas la destruction d'un embryon humain, comme par exemple celui décrit dans WO 2003/046141.

Les cellules cutanées selon la description, qu'elles proviennent d'une biopsie ou qu'elles soient obtenues par différentiation de cellules souches, comprennent au moins un type de cellules habituellement présentes dans l'hypoderme, le derme et/ou l'épiderme. Ces cellules comprennent ainsi, entre autres, des kératinocytes, des mélanocytes, des fibroblastes, des adipocytes, des cellules endothéliales, des mastocytes, des cellules de Langerhans et/ou des cellules de Merkel. De façon préférentielle, les cellules cutanées selon la description comprennent au moins des kératinocytes et/ou des fibroblastes. De façon plus préférentielle, les cellules cutanées selon la description comprennent des kératinocytes et/ou des fibroblastes.

De nombreuses méthodes de culture de cellules cutanées sont connues de la personne du métier. N'importe laquelle de ces méthodes peut être utilisée pour cultiver le substrat cutané de la description. Le substrat cutané est cultivé et/ou conservé dans des conditions maintenant, au moins partiellement, un métabolisme cellulaire et/ou des fonctions cellulaires. La culture du substrat cutané comprend donc aussi bien les cultures de cellules cutanées en monocouche (par ex. de kératinocytes) ou les cultures de cellules cutanées en bicouche que des modèles tissulaires, dont les cultures de peaux reconstruites.

Les cultures de cellules cutanées en monocouche ou en bicouche sont connues et utilisées depuis très longtemps. Par ailleurs, de nombreux modèles tissulaires, dont en particulier des modèles de peaux reconstruites (Rosdy et al., In Vitro Toxicol., 10(1) : 39-47, 1997 ; Ponec et al., J Invest Dermatol., 109(3) : 348-355, 1997 ; Ponec et al., Int J Pharm., 203(1-2) : 211-225, 2000 ; Schmalz et al., Eur J Oral Sci., 108(5) : 442-448, 2000 ; Black et al., Tissue Eng, 11(5-6) : 723-733, 2005 ; Dongari-Batgtzoglou et Kashleva, Nat Protoc, 1(4) : 2012-2018, 2006 ; Bechtoille et al., Tissue Eng, 13(11) : 2667-2679, 2007 ; Vrana et al., Invest Ophthalmol Vis Sci, 49(12) : 5325-5331, 2008 ; Kinicoglu et al., Biomaterials, 30(32) : 6418-6425, 2009 ; Auxenfans et al., Eur J Dermatol, 19(2) : 107-113, 2009 ; Kinicoglu et al., Biomaterials, 32(25) : 5756-5764, 2011 ; Costin et al., Altern Lab Anim, 39(4) : 317-337, 2011 ; Auxenfans et al., J Tissue Eng Regen Med, 6(7) : 512-518, 2012 ; Lequeux et al., Skin Pharmacol Physiol, 25(1) : 47-55, 2012 ; EP 29 678 ; EP 285 471 ; EP 789 074 ; EP 1 451 302 B1 ; EP 1 878 790 B1 ; EP 1 974 718 ; US 2007/0148,771 ; US 2010/0,099,576 ; WO 02/070729 ; WO 2006/063864 ; WO 2006/0,63865 ; WO 2007/064305) sont à la disposition de la personne du métier et sont compris dans la portée de la description.

Préférablement, le modèle de peau reconstruite est sélectionné dans le groupe comprenant les modèles d'épiderme constitué principalement de kératinocytes, les modèles de peau comprenant un derme et un épiderme, et les modèles de peau comprenant un derme, un épiderme et un hypoderme. Les modèles comprenant au moins un derme, forment des tissus de type conjonctifs, tandis que les modèles comprenant au moins un épiderme forment des épithélia stratifiés comprenant les couches caractéristiques du tissu considéré. Par exemple, on peut identifier dans les modèles d'épiderme une couche basale *(stratum basalis),* une couche épineuse *(stratum spinosum),* une couche granuleuse *(stratum granulosum),* et une couche cornée *(stratum corneum).*

Avantageusement, ledit modèle de peau est un modèle d'épiderme comprenant un support matriciel de préférence choisi parmi :
- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, de polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable ;
   Dans ce groupe on trouve les modèles Epiderme reconstruits (Skinethic^{®}) ainsi que le modèle EpiDerm^{®} (Mattek Corporation) ;
- un film ou une membrane à base d'acide hyaluronique et/ou de collagène et/ou de fibronectine et/ou de fibrine.
   Dans ce groupe on peut citer particulièrement les modèles : Laserskin^{®} (Fidia Advanced Biopolymers), Episkin ^{®} (L'Oréal).

Ces modèles peuvent être ensemencés par des fibroblastes dans la partie dermique.

Ces modèles, dans lesquels peuvent être intégrés ou non des fibroblastes, servent de support pour l'ensemencement des kératinocytes et la reconstitution de l'épiderme. Avantageusement, sont introduites, outre les kératinocytes, des cellules pigmentaires, des cellules immunocompétentes, des cellules nerveuses ; de préférence les cellules immunocompétentes sont des cellules de Langerhans.

Il existe par ailleurs des modèles dédiés à la thérapie tissulaire qui peuvent également être utilisés dans le cadre de la présente description. On peut citer les modèles Epidex (Modex Thérapeutiques), Epibase^{®} (Laboratoire Genévrier), Epicell^{™} (Genzyme), Autoderm^{™} et Transderm^{™} (Innogenetics).

Le support matriciel est ensuite ensemencé par des kératinocytes pour reconstruire l'épiderme et obtenir finalement une peau reconstruite.

Avantageusement, le modèle de peau utilisé comprend un modèle dans lequel a été incorporé au moins un type cellulaire complémentaire, comme des cellules endothéliales (CE) et/ou des cellules immunitaires telles que lymphocytes, macrophages, mastocytes, cellules dendritiques et/ou des cellules adipeuses et/ou des annexes cutanées, comme des poils, des cheveux, des glandes sébacées.

Le modèle de peau reconstituée de la description est particulièrement simple à mettre en œuvre. En outre, il ne nécessite pas d'utiliser une lignée cellulaire commerciale particulière, et s'avère avantageusement adaptable.

L'actif ou la formulation peut soit être déposé à la surface du substitut cutané mimant donc une application topique ou soit mis dans le milieu de culture pour mimer une application dite systémique c'est-à-dire similaire à la circulation sanguine.

L'application de l'actif à la surface du substitut cutané peut être direct ou indirect. Dans ce dernier cas en particulier, l'actif peut par exemple être déposé sur un support transparent aux rayonnements testés placé au-dessus du substitut cutané. Une telle application indirecte est notamment utile dans le cas où le substitut cutané est une culture de cellules en monocouche.

Dans un mode de réalisation particulier, l'actif évalué est au moins un filtre solaire. Par filtre solaire, on entend toute substance capable de filtrer le rayonnement solaire pour protéger une surface, typiquement la peau ou les cheveux, d'origine naturelle ou synthétique contre les effets nocifs de ces radiations.

Le filtre solaire est choisi parmi ceux classiquement connus dont :
- les filtres solaires organiques tels que les dérivés de triazine, les dérivés de benzotriazole et phényl benzotriazole, les dérivés du dibenzoylmethane, les dérivés de benzophénone et 2-hydroxybenzophénone amino-substitué, les dérivés de merocyanine, les dérivés de l'acide para aminobenzoïque, les dérivés salicyliques, les dérivés cinnamiques, les dérivés de B,B'-diphénylacrylate, les dérivés de benzylidène camphre, les dérivés de phenylbenzimidazole, les dérivés anthraniliques, les dérivés d'imidazolines, et les dérivés de benzylmalonate ; et
- les filtres solaires inorganiques tels que les oxydes métalliques enrobés ou non comme les oxydes de titane, de fer, de zinc, de zirconium, ou de cérium.

Parmi les filtres organiques usuels, on peut citer en particulier la 5,6,5',6'-tétraphényl-3,3'-(1,4-phénylène)-bis[1,2,4]triazine (n° CAS : 55514-22-2), également appelée phénylène bis-diphényltriazine ; le méthoxypropylamino cyclohexènylidène éthoxyéthylcyanoacétate (nom chimique : 2- éthoxyéthyl (2Z)-2-cyano-2-[3-(3-méthoxypropylamino)cyclohex-2-èn-1-ylidène]acétate ; N° CAS : 1419401-88-9), également appelé S87 ; l'hexyl 2-[4-(diéthylamino)-2-hydroxybenzoyl]benzoate (n° CAS : 302776-68-7), également appelé diéthylamino hydroxybenzoyl hexyl benzoate ; la 2,4-bis[4-(2-éthylhexyloxy)-2-hydroxyphényl]-6-(4-méthoxyphényl)-1,3,5-triazine (n° CAS : 187393-00-6), également appelée bis-éthylhexyloxyphenol méthoxyphenyl triazine, bemotrizinol ou anisotriazine ; le 4-tert-butyl-4-méthoxydibenzoylmethane ou butyl méthoxydibenzoylméthane (nom chimique : 1,4-(1,1-diméthyléthyl)phényl-3-(4-méthoxyphényl)-1,3-propanedione ; n° CAS : 70356-09-1), également appelé avobenzone ou BMDBM ; l'éthylhexyl triazone (nom chimique : acide 4-[[4,6-bis[[4-(2-éthylhexoxy-oxométhyl)phényl]amino]-1,3,5-triazin-2-yl]amino]benzoïque 2-éthylhexyl ester ; n° CAS : 88122-99-00) ; la diéthylhexyl butamido triazone (nom chimique : 4,4'-[[6-[[4-[[(1,1-diméthyléthyl)amino]carbonyl]phényl]amino]-1,3,5-triazine-2,4-diyl]diimino]bis-, bis(2-éthylhexyl)benzoate ; n° CAS : 154702-15-5) ; le méthylène bis-benzotriazolyl tétraméthylbutylphenol (nom chimique : 2,2'-méthylène-bis-(6-(2H-benzotriazol-2-yl)-4-(1,1,3,3-tetraméthylbutyl)phénol ; n° CAS : 103597-45-1), également appelé MBBT ; la 2,4,6-tris(biphényl-4-yl)-1,3,5-triazine (n° CAS : 31274-51-8), également tris-biphényl triazine.

L'actif peut être le filtre solaire seul ou formulé dans une composition à usage topique. On peut bien entendu également tester des combinaisons de filtres solaires, les protections solaires comprenant usuellement un mélange de filtres solaires afin d'offrir un spectre de protection le plus large possible.

On pourrait également envisager que l'actif soit un produit physique, tel que par exemple un textile porté par le sujet ou un parasol ou toute autre toile sous lequel le sujet s'abrite.

Dans la méthode selon la description, le rayonnement est plus particulièrement un rayonnement solaire.

Le rayonnement susceptible d'induire des dommages de l'ADN peut être un rayonnement ultraviolet solaire ou artificiel,

Préférentiellement, le rayonnement susceptible d'induire des dommages de l'ADN est un rayonnement mimant un rayonnement solaire, plus préférentiellement une irradiation dans le domaine UV dont UVA et/ou UVB et/ou UVC ; et/ou lumière bleue haute énergie visible et/ou infrarouge. Les UVC sont naturellement arrêtés par la couche d'ozone de l'atmosphère mais sont régulièrement utilisés par des industries à des fins de stérilisation ou d'élimination de pathogènes.

Par « rayonnement ultraviolet » on entend des rayons électromagnétiques dont la longueur d'onde est comprise de 100 nm à 400 nm. Préférentiellement, l'exposition aux UV selon la description comprend l'exposition à des UVA et/ou à des UVB. Par UVA, on entend au sens de la description des rayonnements dont la longueur d'onde est comprise de 320 à 400 nm. Par UVB, on entend au sens de la description des rayonnements dont la longueur d'onde est comprise de 280 à 320 nm. Par UVC, on entend au sens de la présente description des rayonnements dont la longueur d'onde est comprise entre 100 à 280 nm. Par « lumière visible » on entend des rayonnements dont la longueur d'onde est comprise de 400 à 700 nm. Par « lumière bleue haute énergie visible » on entend la lumière visible avec une longueur d'onde compris de 400 à 450 nm. Par « infrarouge » on entend des rayonnements dont la longueur d'onde est comprise de 700 nm à 2500 nm.

Selon un mode de réalisation préféré, l'exposition aux UV selon la description comprend ou consiste en l'exposition à des UVA. Selon un autre mode de réalisation préféré, l'exposition aux UV selon la description comprend ou consiste en l'exposition à des UVB. Selon un mode de réalisation avantageux, l'exposition aux UV selon la description comprend ou consiste en l'exposition à des UVA et à des UVB. Selon un autre mode de réalisation avantageux, l'exposition aux rayonnements selon la description comprend ou consiste en l'exposition à des UVA et/ou UVB et/ou UVC et/ou lumière bleue haute énergie visible et/ou IR. Dans un mode de réalisation particulier, les longueurs d'onde appliquées sont comprises entre 280 et 450 nm ou entre 280 à 400 nm. Dans un autre mode de réalisation particulier, la dose d'UVB est comprise entre 80 et 150 mJ/cm2 d'UVB ; de préférence 120 mJ/cm2 d'UVB. La réalisation d'une irradiation peut être effectuée dans des conditions bien connues de la personne du métier (cf. par ex. Iordanov et al, J. Biol. Chem. 1998).

Il peut être avantageux, dans le cadre de la méthode d'évaluation de l'actif, de comparer le substitut cutané traité avec une référence. Dans un mode de réalisation préféré, la méthode d'évaluation de l'actif comprend donc une étape de comparaison de la concentration des dimères de pyrimidines mesurée à l'étape c) dans au moins un milieu de culture du substitut cutané avec une référence.

Cette référence peut être notamment la concentration des dimères de pyrimidines dans un substitut cutané qui n'a pas été mis en contact avec l'actif et/ou dans le milieu de culture de ce substitut cutané. Préférablement, la référence correspond à la concentration des dimères de pyrimidines dans le milieu de culture d'un substitut cutané qui n'a pas été mis en contact avec l'actif. Ce peut aussi être la concentration des dimères de pyrimidines dans le substitut cutané avant la mise en contact avec l'actif et/ou dans le milieu de culture de ce substitut cutané. Préférablement, la références est alors la concentration des dimères de pyrimidines dans le milieu de culture du substitut cutané avant la mise en contact avec l'actif.

Dans ce cas, l'actif évalué possède un pouvoir photoprotecteur si la concentration de dimères de pyrimidines mesurée pour cet actif est inférieure ou égale à celle mesurée pour la référence.

Les termes « inférieur à » ou « diminué », tel qu'utilisés ici, signifient une moins grande quantité, par exemple, une quantité légèrement inférieure à la quantité d'origine, ou par exemple une quantité fortement réduite par rapport à la quantité d'origine, et notamment toutes les quantités dans l'intervalle. En variante, « diminution » peut faire référence à une quantité ou une activité qui est au moins 1 %, 2 %, 3 %, 4 %, 5 %, 6 %, 7 %, 8 %, 9 %, 10 %, 11 %, 12 %, 13 %, 14 %, 15 %, 16 %, 17 %, 18 %, 19 % ou 20 % de moins que la quantité ou de l'activité pour laquelle la quantité ou de l'activité diminuée est comparé, ou encore au moins 30 %, 40 %, 50 %, 60 %, 70 %, 80 %, 90 %, 100 %, 110 %, 120 %, 130 %, 140 %, 150 %, 160 %, 170 %, 180 %, 190 %, 200 %, 250 %, 300 %, 350 %, 400 %, 450 %, 500 %, 550 %, 600 %, 650 %, 700 %, 750 %, 800 %, 900 %, 950 %, 1 000 %, 1 100 %, 1 200 %, 1 300 %, 1 400 %, 1 500 %, 1 600 %, 1 700 %, 1 800 %, 1 900 % ou 2 000 % de moins que la quantité ou de l'activité pour laquelle la quantité ou de l'activité diminuée est comparé. Les termes « diminué », « plus petit que », et « réduit » sont utilisés ici de manière interchangeable.

La référence peut aussi consister en la concentration des dimères de pyrimidines dans un substitut cutané qui a été mis en contact avec un actif possédant une activité photoprotectrice avérée et/ou dans le milieu de culture de ce substitut cutané. Préférablement, la référence est dans ce cas la concentration des dimères de pyrimidines dans le milieu de culture d'un substitut cutané qui a été mis en contact avec un actif possédant une activité photoprotectrice avérée. Cet actif photoprotecteur est avantageusement un filtre solaire ou une combinaison de filtres solaires pouvant être formulé avec des excipients usuellement introduits dans une composition solaire.

Dans ce cas, l'actif évalué possède un pouvoir photoprotecteur si la concentration de dimères de pyrimidines mesurée pour cet actif est supérieure ou égale à celle mesurée pour la référence.

Les termes « supérieur à » ou « augmenté », tel qu'utilisés ici, signifient une plus grande quantité, par exemple, une quantité légèrement supérieure à la quantité d'origine, ou par exemple une quantité en grand excès par rapport à la quantité d'origine, et notamment toutes les quantités dans l'intervalle. En variante, « augmentation » peut faire référence à une quantité ou une activité qui est au moins 1 %, 2 %, 3 %, 4 %,5 %, 6 %, 7 %, 8 %, 9 %, 10 %, 11 %, 12 %, 13 %, 14 %, 15 %, 16 %, 17 %, 18 %, 19 % ou 20 % de plus que la quantité ou de l'activité pour laquelle la quantité ou de l'activité accrue est comparé, ou encore au moins 30 %, 40 %, 50 %, 60 %, 70 %, 80 %, 90 %, 100 %, 110 %, 120 %, 130 %, 140 %, 150 %, 160 %, 170 %, 180 %, 190 %, 200 %, 250 %, 300 %, 350 %, 400 %, 450 %, 500 %, 550 %, 600 %, 650 %, 700 %, 750 %, 800 %, 900 %, 950 %, 1 000 %, 1 100 %, 1 200 %, 1 300 %, 1 400 %, 1 500 %, 1 600 %, 1 700 %, 1 800 %, 1 900 % ou 2 000 % de plus que la quantité ou de l'activité pour laquelle la quantité ou de l'activité accrue est comparé. Les termes « augmenté », « plus grand que », et « accru » sont utilisés ici de manière interchangeable.

Dans un autre aspect, la description permet d'identifier des actifs ayant une activité photoprotectrice.

Selon un autre aspect, la description porte donc sur une méthode d'identification d'un actif photoprotecteur, ladite méthode comprenant :
a) L'évaluation de l'activité photoprotectrice de l'actif selon la méthode décrite plus haut, et
b) L'identification de l'actif comme un actif photoprotecteur si l'activité photoprotectrice évaluée dans l'étape a) est supérieure à une référence.

Préférentiellement, la référence est l'activité photoprotectrice d'un actif photoprotecteur connu évaluée selon la méthode décrite plus haut.

L'actif photoprotecteur est avantageusement un filtre solaire ou une combinaison de filtres solaires pouvant être formulé avec des excipients usuellement introduits dans une composition solaire.

La description sera décrite de manière plus précise à l'aide des exemples ci-dessous.

### EXEMPLES

La formulation suivante a été testée dans l'exemple ci-après.

**Tableau 1 : Formulation A**

| Ingrédients | % (m/m) |
|---|---|
| 5,6,5',6'-tetraphényl-3,3'-(1,4-phénylène)-bis[1,2,4]triazine | 3-3,5 |
| Bis-Ethylhexyloxyphenol Méthoxyphényl Triazine | 2,5-3 |
| Diéthylamino hydroxybenzoyl hexyl benzoate | 5-6 |
| Ethyl hexyl Triazone | 3,5-4 |
| Glycérine | 5,0 |
| Eau déminéralisée | Qsp 100 |
| Gomme xanthane | 0,2 |
| C12-C15 alkyl benzoate | 12,0 |
| Dicaprylyl carbonate | 9,0 |
| Triglycérides caprylique/caprique | 9,0 |
| Conservateurs | qs |
| VP / eicosène copolymère | 1,0 |
| Cétyl phosphate de potassium | 2,0 |

Le but de cette étude est d'évaluer les effets photoprotecteurs de formulations sur les dommages de l'ADN photoinduits à partir de prélèvements épidermiques après exposition solaire chronique en condition réelle chez des adultes sains. L'approche a été validée avec un produit de formule A.

### Matériaux et méthodes

### Produits chimiques et enzymes

### Réactifs

Le méthanol (MeOH) > 99,9 % de pureté (qualité CHROMASOLV) a été acheté chez Sigma-Aldrich et l'acétonitrile (ACN) de qualité LCMS chez Carlo Erba. L'acétate de triéthylammonium (TEAA, 1 M dans l'eau) et la 1,*N*⁶-éthéno-2'-désoxyadénosine (εdAdo) provenaient de Sigma (Saint-Quentin- Fallavier, France). Les réactifs et solvants pour la synthèse (5'-diméthoxytrityl 3'-phosphoramidite-thymidine, chlorure de diméthoxytrityle, anhydride acétique, acide acétique, pyridine, éthyl-S-triazol, iode, hydroxyde d'ammonium) étaient de la plus haute pureté disponible et achetés chez Aldrich (Saint-Quentin- Fallavier, France). La (¹³C₁₀, 98 % ¹⁵N₂, 96-98 %)-thymidine provenait de Cambridge Isotope Laboratories. La [¹⁵N₅]εdAdo a été synthétisé comme indiqué précédemment (Douki et al. Radic libre Biol Med. 37:62-70, 2004). La thymidylyl-(3'-5')-thymidine (TpT) a été préparée par synthèse de phosphodiester et tous les photoproduits de dinucléosides monophosphates ont été obtenus comme indiqué précédemment (Douki et al. Biochimie. 40:2495-501, 2001).

### Oligonucléotides synthétiques

Les oligonucléotides (qualité purification HPLC) ont été achetés chez Eurogentec (Angers, France). Les oligonucléotides irradiés aux UV (oligo-UV) ont été préparés par irradiation UV (UVC, 30 min) de solutions (0,2 mg.mL-¹⁾ d'un 15-mer (SEQ ID NO :1 : CATCTTACATTTTAC) et d'un 5-mer (SEQ ID NO :2 : TCTTA). Les mélanges résultants ont été calibrés pour leur teneur en photoproduits par HPLC-MS/MS après hydrolyse enzymatique comme détaillé plus bas. Lorsqu'elles sont utilisées dans des échantillons de contrôle qualité (CQ), les deux solutions sont mélangées. Pour la synthèse d'oligonucléotides portant des bases éthenoadénine (oligo-εdAdo), des solutions d'un 5-mer (SEQ ID NO :3 : ATTGC), d'un 10-mer (SEQ ID NO :4 : TATTGTTGCA), d'un 15-mer (SEQ ID NO:5: CATTGTATTGTTGCA) et d'un 20-mer (SEQ ID NO:6: ATTGTATTGTATTGATTGCAA) ont été préparées individuellement en concentration correspondant à 0,1 mM d'adénine. Les solutions (500 ul chacune) ont été mélangées et traitées pendant une nuit avec du chloroacétaldéhyde (10 mM). Les oligonucléotides ont été purifiés et la solution a été calibrée pour la teneur en εdAdo par HPLC-MS/MS.

### Synthèse de [M+12]-photoproduits

Nous avons d'abord synthétisé un dérivé marqué [M+12] de TpT. La première étape était la condensation de [¹³C₁₀,¹⁵N_{2]}-thymidine protégée à l'extrémité 3' en un synthon phosphoramidite de thymine à l'extrémité 5'. Le composé résultant a été oxydé en dinucléoside monophosphate et déprotégé dans des conditions acides et alcalines. La fraction soluble dans l'eau du mélange réactionnel a été purifiée par HPLC en phase inverse. Le produit isolé a été caractérisé par fragmentation de spectrométrie de masse par comparaison avec du TpT non marqué. Tous les fragments étaient identiques dans les deux cas à l'exception des déplacements de m/z expliqués par la présence d'atomes de ¹³C ou de ¹⁵N. Une solution aqueuse de [M+12]-TpT a ensuite été exposée aux UVC pour donner des photoproduits dimériques. Le rendement de modification de [M+12]-TpT a été surveillé au cours de l'irradiation et celle-ci a été arrêtée lorsque le rendement de TT CPD avait atteint un plateau à la suite de la-réversion induite par les UVC. La solution irradiée a ensuite été purifiée sur un système HPLC en phase inverse avec détection UV (230 nm) et la fraction contenant les photoproduits a été isolée. La teneur en TT CPD et 64PP a été déterminée par HPLC-MS/MS avec détection en mode MRM. Un étalonnage externe utilisant des standards non marqués a été utilisé.

### Enzymes

La protéinase K a été achetée auprès de Qiagen (Courtaboeuf, France). La phosphodiestérase II provenait de Worthington (Lakewood, NJ). La RNase A, la DNase II, la nucléase P1, la phosphatase alcaline et la phosphodiestérase I ont été achetées chez Sigma (Saint-Quentin- Fallavier, France).

### Traitement des échantillons biologiques

### Extraction en phase solide

Le protocole SPE pour la quantification du TT CPD et du TT 64PP dans les modèles cutanés in vitro comprenait plusieurs étapes. Tout d'abord, l'oligo-εdAdo a été ajouté (2,5 µL, 100 nM εdAdo) à 100 µL de milieu. 10 µL de TEAA 1 M ont été ajoutés, respectivement. L'échantillon a été déposé sur le dessus d'une colonne SPE HRX-100 (granulométrie-45 µm, phase 100 mg) (Macherey Nagel, Gutenberg, France) conditionnée avec une solution aqueuse 50 mM de TEAA. Le liquide a été éliminé sous vide et la colonne lavée avec 1 ml de tampon (15% MeOH + 85% de solution aqueuse de 50 mM TEAA). L'élution a ensuite été effectuée avec 1 mL d'un mélange de MeOH (75 %) et de TEAA 50 mM (25 %). Après évaporation du MeOH dans un speed-vac (modèle SPD 111V, Savant, Thermo Scientific) et de l'eau par lyophilisation dans un système Alpha 1-2 (Christ), le résidu obtenu a été solubilisé dans 100 µL d'une solution contenant les photoproduits [M+12] de TpT (5 nM TT CPD et 3,5 nM TT 64PP) et 5 nM de [M+5]-εdAdo.

### Extraction et hydrolyse de l'ADN

L'ADN est extrait des cellules HaCat comme décrit précédemment (Ravanat et al. (2002) Carcinogenesis 23 : 1911-1918). Le culot cellulaire subit d'abord une lyse dans un tampon contenant du triton X-100. Les noyaux sont isolés par centrifugation et lysés dans un second tampon contenant du SDS. L'ADN est précipité par un mélange d'iodure de sodium et d'isopropanol. L'ADN est extrait des explants et des épidermes reconstruits en utilisant le kit DNeasy de Qlagen. Les échantillons sont mécaniquement broyés avec des billes d'acier dans un TissueLyzer (Qiagen) en présence de tampon AL (Qiagen). La protéinase K est ajoutée et les échantillons sont incubés à 55°C pendant 3h. L'ARNase est ajoutée et les échantillons sont laissés à température ambiante pendant 2 minutes. Puis du tampon ATL (Qiagen) est ajouté et les échantillons sont placés à 70°C pendant 10 minutes. Après refroidissement, de l'éthanol est ajouté et les échantillons sont vortéxés. Les solutions sont ensuite déposées sur des colonnes DNeasy (Qiagen). La phase liquide est éliminée par centrifugation. Les colonnes sont lavées successivement avec des tampons AW1 et AW2 (Qiagen) par ajout sur la colonne puis centrifugation. L'ADN est obtenu en solution aqueuse par élution de la colonne par de l'eau Milli-Q. L'ADN est ensuite hydrolysé par incubation avec un mélange de phosphodiestérase II, DNAse II et nucléase P1 à pH 6 pendant 2 heures à 37°C. Le pH est ajusté à 8 et une seconde incubation avec de la phosphodiestérase I et de la phosphatase alcaline est faite pendant 2 heures à 37°C. Une solution d'HCl 0,1 N est ajoutée. Les échantillons sont filtrés et transférés dans des flacons d'injection HPLC.

### Isolement des fragments oligonucléotides, des fractions cytoplasmiques et des milieux de culture

Après irradiation des cellules HaCat, le milieu est collecté et un culot cellulaire préparé. Chaque culot de cellules HaCat est divisé en deux fractions égales. L'une est utilisée pour l'extraction de l'ADN comme décrit ci-dessus et l'autre pour l'isolement des fragments oligonucléotides de la fraction cytoplasmique. A cette fin, une lyse des cellules est réalisée avec un tampon HEPES (200 µL) contenant MgCl₂, KCl et 0,01% de Triton X-100. Les cellules sont lysées par une série d'étapes de congélation/décongélation. La solution obtenue est filtrée d'abord à travers une membrane en nylon de 0,2µm (centrifugation à ultrafiltration, VWR) avant utilisation.

### Hydrolyse enzymatique d'oligonucléotides isolés ou d'ADN extrait.

Les solutions d'oligonucléotides purifiés par SPE ou d'ADN nucléaire extrait ont été hydrolysées par voie enzymatique en deux étapes comme décrit précédemment (Courdavault et al. Photochem Photobiol. 79:145-51, 2004). En bref, l'échantillon a d'abord été incubé pendant 2 h à pH 5,5 avec la nucléase P1, la phosphodiestérase II et la DNase II. Le pH a été élevé à 8 par addition de tampon Tris et l'hydrolyse a été reprise pendant 2h en présence de phosphatase alcaline et de phosphodiestérase I. La solution résultante a été filtrée à 0,2 µm et transférée dans des flacons HPLC. Des échantillons de contrôle qualité (QC) pour les analyses de milieu ont été préparés en mélangeant oligo-εdAdo (2,5 µL, 100 nM) à une solution d'oligo-UV (2,5 µL à 100 nM dans TT CPD et 52 nM dans TT 64PP). Le mélange a été lyophilisé et traité en utilisant le protocole appliqué aux échantillons de milieu purifiés par SPE.

### Analyses par chromatographie liquide-spectrométrie de masse en tandem

### Préparation de la courbe d'étalonnage

Les échantillons pour la courbe d'étalonnage ont été préparés en mélangeant des volumes croissants (0, 1, 2 et 5 µL) d'un mélange de photoproduits TpT à 100 nM et d'une solution de εdAdo (0, 1, 2 et 5 µL à 100 nM) dans des flacons HPLC. Les échantillons ont ensuite été lyophilisés-et solubilisés dans 100 µL de la solution d'étalon interne isotopiquement marqué (50 nM de photoproduits [M+12] de TpT et 50 nM [¹⁵N₅]-εdAdo. La même solution a été utilisée pour la solubilisation des-échantillons purifiés par SPE et du CQ. Pour les mesures dans l'ADN, les standards des photoproduits TT, TC et CT (0, 1, 2 et 5 µL à 100 nm) ont mélangés avec 50 nM de photoproduits [M+12] de TpT. La calibration est faite en étalon interne.

### Chromatographie liquide

Les séparations chromatographiques ont été effectuées sur un système chromatographique de la série ExionLC AD (SCIEX, Framingham, MA) équipé d'une colonne SPE en ligne en gel de silice octadécylsilyle (Nucleodur C18 Gravity, granulométrie 5 µm, ID 2 × 50 mm, Macherey-Nagel, Gutenberg, France) et une colonne analytique de gel de silice avec un greffage mixte pentafluorophényl (PFP) / C18 (Excel C18-PFP, granulométrie 1,7 µm, ID 2,1 × 100 mm, ACE, Aberdeen, Royaume-Uni). La-partie SPE en ligne du système consistait en deux pompes, un injecteur automatique et une vanne à 6 voies située dans le four à colonne (température 30°C). La colonne C18 SPE en ligne était connectée à la sortie de l'injecteur automatique et à une entrée de la vanne 6 voies. Le gradient variait d'abord de 0 à 10 % d'ACN dans 20 mM de TEAA en 1 min à un débit de 400 µL min-^{1.} La vanne 6-voies commutait de la position "load" à la position "inject" 1 min après l'injection de l'échantillon. Dans cette configuration, la phase mobile dans la colonne SPE était fournie par deux pompes de la partie analytique du système chromatographique. Après 4 min, la vanne a été remise en position "load", et la colonne SPE était rincée par 50% d'ACN entre 6 et 8 min avant d'être équilibrée dans du TEAA pur 20 mM. Pendant ce temps, l'échantillon purifié était élué dans la colonne analytique PFP-C18. Le débit était de 200 µL min⁻¹. Le solvant A était 5 mM de TEAA et le solvant B 20 % d'ACN dans 5 mM de TEAA. Le gradient était laissé à 10 % de B pendant 1 minute puis augmenté linéairement à 45 % jusqu'à 9 min. La proportion de B était ensuite augmentée jusqu'à 100 % en 7 min et la phase mobile a maintenue à cette composition pendant 1 min. A la sortie de la colonne d'analyse, le flux traverse un détecteur UV à barrette de diodes (220-320 nm) utilisé pour quantifier les nucléosides normaux dans les échantillons d'ADN par étalonnage externe. La sortie était alors dirigée vers le spectromètre de masse.

### Spectrométrie de masse

Le spectromètre de masse était un appareil 6500+ QTrap (SCIEX, Framingham, MA). Dans la première partie de l'analyse (9 min), il a été opéré en mode d'ionisation électrospray négatif pour la détection des photoproduits. Ensuite, la polarité a ensuite été changée en positive jusqu'à la fin de la course (20 min) pour la détection de εdAdo. Les paramètres de la source ont été optimisés pour atteindre le meilleur compromis pour les différents analytes. En mode négatif, la pression d'azote du gaz rideau, du gaz source d'ions 1 (nébulisation) et du gaz source d'ions 2 (gaz de gaine chauffé) ont été réglées à 45, 60 et 60 psi respectivement. Le gaz de collision a été réglé sur High. La tension d'ionisation électrospray était de-4300 V et la température de la source de 550°C. Le gaz d'exhaust (air) était réglé à 25 L min⁻¹. Le dwell time était de 50 ms pour toutes les transitions. Pour la détection en ionisation positive de εdAdo, le gaz rideau, le gaz source d'ions 1, le gaz source d'ions 2, le gaz de collision, la tension d'ionisation électrospray et la température de la source ont été réglés sur 45 psi, 60 psi, 60 psi, High, 5000 V et 550° C, respectivement. Au cours de cette période, le dwell time était de 400 ms. La détection a été faite en « multiple reaction monitoring » (MRM) dans lequel le signal correspondant à des réactions de fragmentation spécifiques à chaque analyte sont quantifiés. Trois transitions MRM ont été utilisées pour les photoproduits dimères et une seule pour εdAdo qui ne subit que la perte de l'unité désoxyribose. (Tableau 2). Seuls le TT CPD et le TT 64PP ont été quantifiés dans les échantillons de milieu.

**Tableau 2 : Caractéristiques spécifiques des analytes dans le dosage UHPLC-MS/MS. Rt est le temps de rétention (en min). Les MRM sont les réactions de fragmentation. La première valeur est le rapport m/z de l'ion pseudo-moléculaire ([M-H-]⁻ et [M+H] ⁺en mode négatif et positif, respectivement). La deuxième valeur est le rapport m/z des fragments suivis. La valeur entre parenthèses est l'énergie de collision. Les étalons internes (IS) sont les photoproduits [M+12] de TpT et [M+5]-εdAdo.**

| IDENTIFIANT | Rt | MRM 1 | MRM 2 | MRM 3 |
|---|---|---|---|---|
| TT DPC | 6.3 | 545,2 →79,1 (-130) | 545,2 →195,1 (-47) | 545,2 →447,1 (-42) |
| TT 64PP | 6.9 | 545,2 →79,1 (-130) | 545,2 →195,1 (-47) | 545,2 →432,1 (-36) |
| TC DPC | 4.6 | 531,2 →79,0 (-130) | 531,2 →195,0 (-44) | 531,2 →433,1 (-40) |
| CC DPC | 5.2 | 517,2 →79,0 (-130) | 517,2 →195,0 (-40) | 517,2 →419,1 (-40) |
| TT CPD IS | 6.3 | 557,2 →79,1 (-130) | 557,2 →195,1 (-47) | 557,2 459,1 →(-42) |
| TT 64PP IS | 6.7 | 557,2 →79,1 (-130) | 557,2 →195,1 (-47) | 557,2 →444,1 (-36) |
| εdAdo | 10.6 | 276,0 160,0 →(19) | | |
| εdAdo IS | 10.6 | 281,0 →165,0 (19) | | |

### Expression des résultats

Les courbes d'étalonnage et la correction avec le standard interne oligo-εdAdo ont fourni des concentrations précises exprimées en nM de la solution injectée. Dans l'ADN, εdAdo n'a pas été utilisé. La normalisation à la quantité d'ADN extrait a plutôt été obtenue à partir de la quantité de bases normales quantifiées par un détecteur UV placé devant l'entrée du spectromètre de masse. Les résultats ont ensuite été exprimés en nombre de dimères par million de bases normales.

### Modèles cutanés in vitro

Les cellules HaCat ont été cultivées dans un milieu DMEM GluMAX^{™} (Gibco) complété par 10% (v/v) de sérum de veau fœtal (Gibco). Un mélange d'antibiotiques (50 U/ml de pénicilline et 50 mg/ml de streptomycine) a été ajouté dans une proportion de 1% (v/v). Les cellules ont été ensemencées à 3x 10⁵ ml⁻¹ dans des boites de Pétri de 35 mm de diamètre avec un milieu de 2ml. Ils ont été cultivés dans un incubateur (Hera Cell, Heraeus) à 37°C dans une atmosphère à 5% de CO₂ jusqu'à 70% de confluence et utilisés pour l'irradiation.

Des échantillons de peau humaine ont été prélevés à la suite d'une chirurgie mammaire sur des donneuses en bonne santé avec leur consentement éclairé. Tous les donneurs étaient de phototype de peau II ou III, selon l'échelle de classification de Fitzpatrick. Immédiatement après la collecte, une couche épidermique de 0,25 mm d'épaisseur a été isolée à l'aide d'un dermatome portatif SOBER (Humeca, Enschede, Pays-Bas). Des biopsies à l'emporte-pièce de douze mm ont ensuite été préparées avec des poinçons stériles 5help Medical, France). Ils ont été placés avec le côté dermique vers le bas dans des inserts ThinCert^{™} (Greiner Bio-One, Autriche) maintenus dans 12 plaques de culture cellulaire de puits (Greiner Bio-One, Autriche). Les explants ont été maintenus à l'interface liquide/air en milieu DMEM/F-12 (Thermo-Fischer) contenant 10% de PenStrep à 37°C dans un incubateur humidifié contenant 5% de CO₂. L'épiderme reconstruit humain est produit à partir de kératinocytes normaux primaires humains après 8 jours d'exposition à l'air sur une membrane en polycarbonate inerte. Les kératinocytes ont été isolés à partir de dermolipectomie abdominale et obtenus chez 3 sujets sains caucasiens sans antécédents de maladie de peau et avec leur consentement éclairé.

### Irradiations

Toutes les irradiations ont été réalisées avec une lumière solaire simulée qui correspond à un spectre présentant 5% d'UVB et 95% d'UVA, représentatif de la proportion naturelle UVB/UVA. Les sources d'UV utilisées étaient soit un simulateur LS1000 (Solar Light Company, Glenside, PA) pour les cellules et les explants, soit une chambre Suntest CPS+ (ATLAS) équipée d'une lampe au xénon NXE1500 et équipée d'un filtre UV pour éliminer les longueurs d'onde inférieures à 290 nm pour les épidermes reconstruits humains. En effet un filtre en dessous de 290 nm permet d'éviter d'avoir des UVC très toxiques qui endommagent l'ADN. Les irradiations ont été effectuées avec les échantillons placés dans du PBS afin d'éviter la photosensibilisation par composant du milieu de culture. Après l'irradiation, des échantillons sont prélevés à différents temps de post-irradiation, 3h, 24h, 48, 72h et 96h, à chaque point, les cellules et les milieux de culture sont collectés.

Toutes les expériences sont réalisées à partir de 3 donneurs indépendants et au moins 3 répliquats.

### Analyses statistiques

Les comparaisons intra-groupe et inter-groupes ont été effectuées à l'aide de modèles linéaires adaptés aux paramètres à analyser et aux nombres de visites. De manière générale, le facteur « groupe » (groupe traité avec Formule A versus groupe Contrôle), a été inclus en effet fixe. A cela, ont été ajoutés le facteur « visite » (V2, V3) et l'interaction groupe*visite, eux-aussi en effets fixes. Le facteur « sujet » a été inclus en effet aléatoire et la valeur V1 à la base en tant que covariable. Tout facteur considéré comme pertinent pour l'analyse (jour d'acquisition, etc.) a été pris en compte dans le modèle en effet fixe, le cas échant. Les comparaisons intra-groupe et inter-groupe ont été réalisées sur les différences de moyennes ajustées données par le modèle.

### Résultats

### Photoproduits d'ADN dans les cellules HaCat

Les cellules HaCat sont soumises à une exposition solaire simulée (SSL) dans le PBS. Le milieu de culture et les cellules sont collectés à différents temps post-irradiation. Le contenu en TT-CPD et TT-64PP est déterminé dans les trois échantillons (ADN de cellules, cytoplasme et milieu de culture). Les inventeurs montrent dans la figure ci-dessous que l'approche développée permet de suivre de façon quantitative, tant pour TT CPD (a) que TT 64PP (b), les photoproduits éliminés de l'ADN par les systèmes de réparation d'abord dans le cytoplasme puis le milieu. Le niveau de photoproduits dans l'ADN nucléaire diminue continuellement pendant la période étudiée, avec moins de 20% de TT CPD restant seulement après 96 heures post-irradiation. Le photoproduit TT 64PP est réparé beaucoup plus efficacement et n'est pas détecté après 24 heures post-irradiation. Une tendance inverse est observée avec le milieu de culture, avec une augmentation constante des deux photoproduits avec le temps. La concentration des photoproduits dans le cytoplasme des cellules présente un profil en cloche. La valeur maximale est obtenue à 24 heures post-irradiation pour les deux photoproduits, puis on observe une diminution progressive.

**Tableau 3**

| **TT CPD** | ADN nucléaire | Cytoplasme | Milieu |
|---|---|---|---|
| Temps (h) | per 10⁶ bases | nM | nM |
| 0 | 14.94±3.90 | 0.08±0.03 | 0.03±0.02 |
| 3 | 12.30±1.61 | 0.23±0.07 | 0.02±0.02 |
| 24 | 5.59±2.83 | 0.37±0.06 | 0.05±0.01 |
| 48 | 4.11±0.27 | 0.25±0.04 | 0.05±0.02 |
| 72 | 3.50±0.42 | 0.16±0.03 | 0.10±0.04 |
| 96 | 2.65±0.75 | 0.10±0.07 | 0.10±0.06 |

| **TT 64PP** | ADN nucléaire | Cytoplasme | Milieu |
|---|---|---|---|
| Temps (h) | per 10⁶ bases | nM | nM |
| 0 | 0.590±0.245 | 0.039±0.004 | 0.004±0.001 |
| 3 | 0.088±0.064 | 0.109±0.025 | 0.003±0.001 |
| 24 | 0.000±0.000 | 0.132±0.034 | 0.019±0.006 |
| 48 | 0.000±0.000 | 0.114±0.018 | 0.037±0.004 |
| 72 | 0.000±0.000 | 0.099±0.021 | 0.082±0.037 |
| 96 | 0.000±0.000 | 0.055±0.014 | 0.092±0.035 |

Les valeurs sont les moyennes ± écart-types (n=6)

### Photoproduits d'ADN dans les explants de peau humaine normale

Des échantillons de peau humaine, sont fraichement obtenus après chirurgie mammaire sur des patientes en bonne santé. Ils sont utilisés pour préparer une série de biopsies *ex-vivo* de 1 cm cultivés à l'interface air/liquide sur des membranes placées dans des puits de plaques en cultures. Les explants sont exposés à des doses croissantes de SSL (2,75 ; 5,5 ; 11 J/cm²). Comme dans le protocole précédent, l'incubation est arrêtée à différents temps de post-irradiation (1, 24 et 96 heures) et les milieux sont collectés. Les inventeurs montrent que les fragments TT CPD et TT 64PP sont détectés après 24 et 96 heures post-irradiation, leur concentration dépend de la dose SSL appliquée. Aucun photoproduit n'est détecté 1 heure après l'irradiation, à l'exception d'une faible quantité de TT 64PP (0,02 ± 0,004 nM) pour 11 J/cm².

### Photoproduits d'ADN dans les épidermes reconstruits

Les épidermes reconstruits sont exposés à des doses croissantes de SSL (2,75 ; 5,5 ; 11 ; 16,5 J/cm²), les épidermes et les milieux à différents temps (6, 24, 48 et 72 heures) post-irradiation sont recueillis.

L'efficacité de réparation évaluée dans l'ADN nucléaire a des rendements d'élimination de 23 et 67% pour les fragments TT CPD et TT 64PP 72 heures après irradiation à 2,75 J/cm². Cependant, les photoproduits sont facilement détectés dans le milieu de culture même pour les plus faibles doses d'irradiation comme on le voit dans le tableau 3.

**Tableau 4 : Concentration (exprimée en nM) de TT CPD et de TT 64PP dans le milieu de culture des épidermes reconstruits**

| | TT CPD | | | |
|---|---|---|---|---|
| Temps post-UV (h) | 2,75 J/cm² | 5,5 J/cm² | 11 J/cm² | 16,5 J/cm² |
| 6 | 0,27 ± 0,02 | 0,58 ± 0,04 | - | - |
| 24 | 2,00 ± 0,22 | 2,94 ± 0,13 | 4,34 ± 0,32 | 5,47 ± 0,20 |
| 48 | 3,37 ± 0,17 | 3,86 ± 0,17 | 6,35 ± 0,37 | 7,52 ± 0,30 |
| 72 | 3,62 ± 0,19 | 4,72 ± 0,16 | 7,55 ± 0,41 | 9,69 ± 0,65 |

| | **TT 64PP** | | | |
|---|---|---|---|---|
| | 2,75 J/cm² | 5,5 J/cm² | 11 J/cm² | 16,5 J/cm² |
| 6 | 0,20 ± 0,04 | 0,47 ± 0,06 | - | - |
| 24 | 1,53 ± 0,39 | 2,15 ± 0,45 | 3,29 ± 0,75 | 3,92 ± 0,87 |
| 48 | 2,27 ± 0,50 | 2,82 ± 0,62 | 4,59 ± 1,07 | 5,42 ± 1,26 |
| 72 | 2,56 ± 0,58 | 3,43 ± 0,77 | 5,29 ± 1,24 | 7,22 ± 1,73 |

Les valeurs représentent les moyennes ± erreurs standards, n=9. Les échantillons à 6 heures n'étaient pas disponibles pour 2 et 3 MED.

Il est donc observé que la concentration des deux fragments TT CPD et TT 64PP augmente en fonction de la dose SSL appliquée et du temps post-irradiation.

Les inventeurs mettent clairement en évidence dans 3 modèles *in vitro* différents qu'il est possible de quantifier dans le milieu de culture, les produits de réparation de l'ADN libérés suite à une irradiation.

### Produits de réparation de l'ADN et photoprotection

Dans cette nouvelle expérience, les inventeurs ont cherché à savoir si l'application d'un photoprotecteur (Formule A) permettait de réduire la concentration des photoproduits quantifiés dans le milieu de culture.

Le choix du modèle s'est porté sur les épidermes reconstruits. Les doses d'irradiation appliquées étaient de 5,5 et 16,5 J/cm².

L'application topique de 2 mg/cm² de Formule A est étalée au pinceau sur les épidermes reconstruits.

Les résultats de cette expérience sont résumés dans le tableau 4 ci-dessous.

**Tableau 5 : Concentration de TT CPD et de TT 64PP dans le milieu de culture (nM) et dans l'ADN (lésions/million de bases) des épidermes reconstruits en présence ou non d'un photoprotecteur (Formule A)**

| SSL | Echantillon | SSL | SSL + Formule A |
|---|---|---|---|
| | TT CPD | | |
| 5,5 J/cm² | ADN | 21 ± 3,2 | 3,3 ± 0,4 |
| | Milieu | 4,1 | 0,5 |
| 16,5 J/cm² | ADN | 89 ± 10 | 11 ± 2 |
| | Milieu | 12,0 | 1,6 |

| | TT 64PP | | |
|---|---|---|---|
| 5,5 J/cm² | ADN | 2,2 ± 0,48 | 0,30 ± 0,09 |
| | Milieu | 0,38 | 0,05 |
| 16,5 J/cm² | ADN | 6,9 ± 0,64 | 0,87 ± 0,21 |
| | Milieu | 0,75 | 0,14 |

Les valeurs des concentrations dans le milieu de culture sont exprimées en nM et correspondent à la somme des données obtenues à 24, 48 et 72h. A la fin de chaque étude cinétique, les concentrations de photoproduits dans le milieu pour les différents temps sont additionnées. Cela permet de s'affranchir de différences éventuelles de cinétique de réparation dues à la photoprotection. Les valeurs des concentrations dans l'ADN sont des moyennes ± erreurs standard de répliquats de l'expérience (n=9).

Les inventeurs mettent donc clairement en évidence que l'application d'un photoprotecteur réduit considérablement la concentration des fragments TT CPD et TT 64PP libérés dans le milieu de culture.

Ces modèles *in vitro* permettent donc d'évaluer l'efficacité d'un photoprotecteur en quantifiant les photoproduits libérés dans les milieux de culture après irradiation.

## Revendications

1. Méthode d'évaluation de l'activité photoprotectrice d'un actif photoprotecteur, la méthode comprenant :
a) la mise en contact de l'actif avec un substitut cutané, puis
b) L'exposition du substitut cutané à un rayonnement, puis
c) Le dosage de dimères de pyrimidine dans le milieu de culture du substitut cutané.

2. Méthode selon la revendication 1, caractérisée en ce le dosage comprend une digestion enzymatique d'acides nucléiques et un dosage des dimères de pyrimidine par chromatographie liquide.

3. Méthode selon l'une quelconque des revendications 1 ou 2, caractérisée en ce le dosage comprend des étapes dans lesquelles :
- les acides nucléiques contenus dans le substitut cutané et/ou dans le milieu de culture du substitut cutané sont extraits en phase solide ; puis
- l'éluat contenant les acides nucléiques obtenu suite à l'étape d'extraction en phase solide est concentré ; puis
- les acides nucléiques contenus dans l'éluat concentré ainsi obtenu sont digérés enzymatiquement ; puis
- les dimères de pyrimidine obtenus suite à l'étape de digestion enzymatique sont dosés par chromatographie liquide.

4. Méthode selon la revendication 2 ou 3, **caractérisée en ce que** le dosage des dimères de pyrimidine comprend la détermination de la concentration des dimères de pyrimidine dans le milieu de culture du substitut cutané, avantageusement par spectrométrie de masse.

5. Méthode selon la revendication 4, **caractérisée en ce qu'**elle comprend une étape supplémentaire de comparaison de la concentration des dimères de pyrimidine dans le milieu de culture du substitut cutané avec une référence.

6. Méthode selon la revendication 5, **caractérisée en ce que** la référence est la concentration des dimères de pyrimidine dans le milieu de culture d'un substitut cutané avec lequel l'actif n'a pas été mis en contact.

7. Méthode selon la revendication 5, **caractérisée en ce que** la référence est la concentration des dimères de pyrimidine dans le milieu de culture du substitut cutané avant la mise en contact avec l'actif ne soit appliqué.

8. Méthode selon l'une quelconque des revendications précédentes, **caractérisée en ce que** les dimères de pyrimidine sont des dimères de thymine, de préférence TT 64PP ou TT CPD.

9. Méthode selon l'une quelconque des revendications précédentes, **caractérisée en ce que** l'actif est au moins un filtre solaire.

10. Méthode selon l'une quelconque des revendications précédentes, **caractérisée en ce que** l'actif est appliqué directement sur le substitut cutané.

11. Méthode selon l'une quelconque des revendications 1 à 10, **caractérisée en ce que** l'actif est ajouté directement dans le milieu de culture.

12. Méthode selon l'une quelconque des revendications précédentes, **caractérisée en ce que** le rayonnement est un rayonnement ultraviolet et/ou lumière bleue haute énergie visible et/ou infrarouge.

13. Méthode d'identification d'un actif photoprotecteur, ladite méthode comprenant :
c) L'évaluation de l'activité photoprotectrice de l'actif selon la méthode de l'une quelconque des revendications 1 à 12, et
d) L'identification de l'actif comme un actif photoprotecteur si l'activité photoprotectrice évaluée dans l'étape a) est supérieure à un seuil.

14. Méthode selon la revendication précédente, **caractérisée en ce que** le seuil est l'activité photoprotectrice d'un actif photoprotecteur connu évaluée selon la méthode de l'une quelconque des revendications 1 à 12.

## Patentansprüche

1. Verfahren zur Beurteilung der Lichtschutzwirkung eines Lichtschutzwirkstoffs, wobei das Verfahren umfasst:
a) das Inkontaktbringen des Wirkstoffs mit einem Hautsubstitut, dann
b) das Exponieren des Hautsubstituts gegenüber einer Strahlung, dann
c) das Bestimmen von Pyrimidindimeren in dem Kulturmedium des Hautsubstituts.

2. Verfahren nach Anspruch 1, **dadurch gekennzeichnet, dass** die Bestimmung einen enzymatischen Verdau von Nukleinsäuren und eine Bestimmung der Pyrimidindimere mittels Flüssigkeitschromatographie umfasst.

3. Verfahren nach einem der Ansprüche 1 oder 2, **dadurch gekennzeichnet, dass** die Bestimmung Schritte umfasst, bei denen:
- die in dem Hautsubstitut und/oder in dem Kulturmedium des Hautsubstituts enthaltenen Nukleinsäuren in der Festphase extrahiert werden; dann
- das im Anschluss an den Schritt der Festphasenextraktion erhaltene, die Nukleinsäuren enthaltende Eluat konzentriert wird; dann
- die in dem so erhaltenen konzentrierten Eluat enthaltenen Nukleinsäuren enzymatisch verdaut werden; dann
- die im Anschluss an den Schritt des enzymatischen Verdaus erhaltenen Pyrimidindimere mittels Flüssigkeitschromatographie bestimmt werden.

4. Verfahren nach Anspruch 2 oder 3, **dadurch gekennzeichnet, dass** die Bestimmung der Pyrimidindimere die Ermittlung der Konzentration der Pyrimidindimere in dem Kulturmedium des Hautsubstituts, vorteilhafterweise mittels Massenspektrometrie, umfasst.

5. Verfahren nach Anspruch 4, **dadurch gekennzeichnet, dass** es einen zusätzlichen Schritt des Vergleichens der Konzentration der Pyrimidindimere in dem Kulturmedium des Hautsubstituts mit einer Referenz umfasst.

6. Verfahren nach Anspruch 5, **dadurch gekennzeichnet, dass** die Referenz die Konzentration der Pyrimidindimere in dem Kulturmedium eines Hautsubstituts ist, mit dem der Wirkstoff nicht in Kontakt gebracht wurde.

7. Verfahren nach Anspruch 5, **dadurch gekennzeichnet, dass** die Referenz die Konzentration der Pyrimidindimere in dem Kulturmedium des Hautsubstituts vor der Anwendung des Inkontaktbringens mit dem Wirkstoff ist.

8. Verfahren nach einem der vorhergehenden Ansprüche, **dadurch gekennzeichnet, dass** die Pyrimidindimere Thymindimere, vorzugsweise TT-64PP oder TT-CPD, sind.

9. Verfahren nach einem der vorhergehenden Ansprüche, **dadurch gekennzeichnet, dass** der Wirkstoff mindestens ein Sonnenfilter ist.

10. Verfahren nach einem der vorhergehenden Ansprüche, **dadurch gekennzeichnet, dass** der Wirkstoff direkt auf das Hautsubstitut aufgetragen wird.

11. Verfahren nach einem der Ansprüche 1 bis 10, **dadurch gekennzeichnet, dass** der Wirkstoff direkt in das Kulturmedium gegeben wird.

12. Verfahren nach einem der vorhergehenden Ansprüche, **dadurch gekennzeichnet, dass** die Strahlung eine ultraviolette Strahlung und/oder hochenergetisches sichtbares blaues und/oder infrarotes Licht ist.

13. Verfahren zur Identifizierung eines Lichtschutzwirkstoffs, wobei das Verfahren umfasst:
c) das Beurteilen der Lichtschutzwirkung des Wirkstoffs nach dem Verfahren nach einem der Ansprüche 1 bis 12, und
d) das Identifizieren des Wirkstoffs als ein Lichtschutzwirkstoff, wenn die in Schritt a) beurteilte Lichtschutzwirkung über einem Schwellenwert liegt*.*

14. Verfahren nach vorhergehendem Anspruch, **dadurch gekennzeichnet, dass** der Schwellenwert die nach dem Verfahren nach einem der Ansprüche 1 bis 12 beurteilte Lichtschutzwirkung eines bekannten Lichtschutzwirkstoffs ist.

## Claims

1. A method for evaluating the photoprotective activity of a photoprotective active ingredient, the method comprising:
a) contacting the active ingredient with a skin substitute, and then
b) exposing the skin substitute to radiation, and then
c) assaying for pyrimidine dimers in the culture medium of the skin substitute.

2. A method according to claim 1, wherein the assay comprises enzymatic digestion of nucleic acids and determination of pyrimidine dimers by liquid chromatography.

3. A method according to any one of claims 1 or 2, wherein the assay comprises steps in which:
- the nucleic acids contained in the skin substitute and/or in the culture medium of the skin substitute are extracted by solid-phase extraction; then
- the eluate containing the nucleic acids obtained following the solid-phase extraction step is concentrated; then
- the nucleic acids contained in the concentrated eluate thus obtained are enzymatically digested; then
- the pyrimidine dimers obtained following the enzymatic digestion step are quantified by liquid chromatography.

4. A method according to claim 2 or 3, wherein the quantification of pyrimidine dimers comprises determining the concentration of pyrimidine dimers in the skin substitute culture medium, preferably by mass spectrometry.

5. A method according to claim 4, wherein it comprises an additional step of comparing the concentration of pyrimidine dimers in the culture medium of the skin substitute with a reference.

6. A method according to claim 5, wherein the reference is the concentration of pyrimidine dimers in the culture medium of a skin substitute with which the active ingredient has not been contacted.

7. A method according to claim 5, wherein the reference is the concentration of pyrimidine dimers in the culture medium of the skin substitute before the active ingredient is applied.

8. A method according to any one of the preceding claims, wherein the pyrimidine dimers are thymine dimers, preferably TT 64PP or TT CPD.

9. A method according to any one of the preceding claims, wherein the active ingredient is at least one sunscreen.

10. A method according to any one of the preceding claims, wherein the active ingredient is applied directly to the skin substitute.

11. A method according to any one of claims 1 through 10, wherein the active ingredient is added directly to the culture medium.

12. A method according to any one of the preceding claims, wherein the radiation is ultraviolet radiation and/or high-energy visible blue light and/or infrared radiation.

13. A method for identifying a photoprotective active ingredient, said method comprising:
a) evaluating the photoprotective activity of the active ingredient according to the method of any one of claims 1 through 12, and
b) identifying the active ingredient as a photoprotective active ingredient if the photoprotective activity evaluated in step (a) exceeds a threshold.

14. A method according to the preceding claim, wherein the threshold is the photoprotective activity of a known photoprotective active ingredient evaluated according to the method of any one of claims 1 through 12.
